# EUROPÄISCHE PATENTSCHRIFT

(11) **EP 1 602 924 B1**
(45) Veröffentlichungstag und Bekanntmachung des Hinweises auf die Patenterteilung: **17.09.2008**
(21) Anmeldenummer: 05011921.3
(22) Anmeldetag: 02.06.2005
(51) Int. Cl.: G01N 33/00, B60H 1/00, G01N 27/12

(54) **Vorrichtung und Verfahren zur Detektion von leichtflüchtigen Organischen Verbindungen**
Apparatus and Method for detecting volatile organic compounds
Dispositif et procédé pour detecter des composés organiques volatiles

(30) Priorität: 03.06.2004 DE 102004027266; 06.12.2004 DE 102004058837
(43) Veröffentlichungstag der Anmeldung: 07.12.2005
(73) Patentinhaber: UST Umweltsensortechnik GmbH, 98716 Geschwenda (DE)
(72) Erfinder: Kiesewetter, Olaf, 98716 Geschwenda (DE); Ewert, Anatolij, 98693 Ilmenau (DE); Melchert Volkmar, 98693 Martinroda (DE); Kittelmann, Sven, 99326 Stadtilm (DE)
(74) Vertreter: Liedtke, Klaus

(56) Entgegenhaltungen:
- EP-A- 0 354 486
- EP-A- 0 833 147
- EP-A- 0 945 722
- DE-A1- 3 833 295
- FR-A- 2 797 498
- US-A- 6 046 054
- HARALD BÖTTNER, JÜRGEN WÖLLENSTEIN: "Millenium Sensor Systems - MISSY" INFOBÖRSE MIKROSYSTEMTECHNIK, Nr. 33, 2002, XP002440651 VDI Germany

## Beschreibung

Die Erfindung betrifft eine Anordnung und ein Verfahren zur Detektion von Luftinbaltsstoffen, vorzugsweise zur Detektion von Luftinhaltsstoffen in Gebäuden und Fahrzeugen.

Zur Detektion von Luftinhaltsstoffen sind verschiedene Ausführungsformen bekannt, bei denen Metalloxid-Gassensoren verwendet werden, welche zur Detektion luftgetragener Schadstoffe geeignet sind.

Es sind Lösungen bekannt, die jeweils für spezielle leichtflüchtige Stoffe (VOC), beispielsweise für Autoabgase, geeignet sind. Es existieren bisher keine Systeme, die in der Lage sind, diese Bereiche gemeinsam abzudecken, um belastungsgerecht z.B. die Klimatisierung in Räumen zu steuern oder Fahrzeugkabinen zu belüften. Da für viele Anwendungsfälle in Fahrzeugen immer weniger Luftdurchsatz möglich ist, werden die bekannten Lösungskonzepte diesen Anforderungen nicht gerecht.

Zur sensorgesteuerten Fahrzeuglüftung sind schon aus den späten 70-er Jahren Lösungen bekannt, die mit Hilfe von Halbleitergassensoren die Klimaanlage steuern.

Aufgrund der stark wechselnden Umgebungsbedingungen wie Temperatur, Feuchte oder andere Einflüsse, beispielsweise Staub, werden zum Regeln oder Steuern relative Pegel verwendet. Wünschenswert wären aber so genannte künstliche Nasen, mit denen sowohl Autoabgase als auch verschiedene Gerüche detektiert werden können, sodass der Sensor sowohl im Außenbereich als auch im Innenbereich verwendet werden kann und eine sensorinterne Wirkschichtüberwachung stattfindet.

Neue Entwicklungen im Bereich der Halbleitergassensoren wie Array-Strukturen, Multichips und dergleichen, ermöglichen die Konstruktion künstlicher Nasen, die es möglich machen, den chemischen Merkmalsraum deutlich besser abzudecken und mit Hilfe neuronaler Netze die Eigenschaften geruchsgeführter Sinnesorgane besser nachzubauen.
Mit diesem System ist es möglich, kleinste Konzentrationen im ppb-Bereich (z.B. Innenbereich Fahrzeug) sowie große Konzentrationen im ppm-Bereich (Außenbereich) zu detektieren. Auch ist die Drift kleiner als bei Einzelsensoren, da kein auf der Schicht überlagertes Signal aus vorhandenen Gasen entsteht. Nachteil dieser sonst sehr vorteilhaften Konzeption ist ihr hoher Preis, weil mehrere Sensoren und eine Vielzahl von elektrischen Anschlussdrähten benötigt werden.

Ein Beispiel für ein System mit mehreren Gassensoren ist das Modul MISSY (Infobörse Mikrosystemtechnik, Nr. 33, 2002).

Der Erfindung liegt die Aufgabe zugrunde, eine Anordnung und ein Verfahren der eingangs genannten Art anzugeben, bei der kostengünstig eine Auswertung mit mehreren sensitiven Schichten möglich ist.

Erfindungsgemäß wird die Aufgabe mit einer Anordnung, welche die in Anspruch 1 angegebenen Merkmale enthält und mit einem Verfahren, welches die in Anspruch 6 angegebenen Merkmale aufweist, gelöst.

Vorteilhafte Ausgestaltungen sind in den Unteransprüchen angegeben.

Die erfindungsgemäße Anordnung ist ein Einzelsensorelement mit vorzugsweise drei für bestimmte Gase sensitiven Wirkschichten, die auswertbare Signale erzeugen, wobei ein chemischer Merkmalsraum komplex erfasst werden kann.

Die Anordnung zeichnet sich durch eine Reihe von Vorteilen aus. Hierzu gehören insbesondere:
- Es können verschiedene Luftinhaltsstoffe erfasst werden;
- die Messung erfolgt mit vorzugsweise drei unterschiedlichen Wirkschichten, wobei nur insgesamt 4 Anschlüssen für die Verbindung der Wirkschichten und eines Heizers mit einer Auswerteeinheit benötigt werden;
- die Anordnung gestattet es, Drift- und Verschmutzungseffekte der Wirkschichten zu erfassen;
- die Anordnung kann sehr kostengünstig hergestellt und beispielsweise in einem Standardgehäuse untergebracht werden;
- die Wirkschichten können vertikal oder horizontal angeordnet sein.

Die Erfindung wird im Folgenden anhand von Ausführungsbeispielen zur Steuerung für das belastungsgerechte Lüften von Kabinen für Kraftfahrzeuge näher erläutert. Die beschriebenen Beispiele sind auch für Anwendungen in anderen geschlossenen Räumen geeignet.

In der zugehörigen Zeichnung zeigen:
- Figur 1: die elektrische Schaltung der Sensoranordnung,
- Figur 2: eine Ausführungsform mit drei übereinander angeordneten Wirkschichten,
- Figur 3: eine Ausführungsform mit drei nebeneinander angeordneten Wirkschichten,
- Figur 4: eine Ausführungsform mit drei versetzt übereinander angeordneten Wirkschichten,
- Figuren 5 und 6: eine Ausführungsform, bei der die Elektroden in unterschiedlichen Abständen angeordnet sind und die sensitiven Elemente in einer aus unterschiedlich dotierten Wirkschichtbereichen bestehenden Schicht integriert sind,
- Figur 7: die Draufsicht auf eine auf einen Chip angeordnete Anordnung,
- Figuren 8 bis 10: den zeitlichen Verlauf verschiedener Betriebsweisen der Anordnung,
- Figur 11: das Schaltbild für eine Anordnung mit Auswerteeinheit
und die
- Figuren 12 und 13: Messwertverläufe, die an verschiedenen Gasproben gewonnen wurden.

Die Anordnung ist auf einem Substrat 1 in einem Standardgehäuse angebracht. Die Auswertung der Signale erfolgt mit einer Auswerteeinrichtung mittels eines vorgegeben Auswertealgorithmus oder mit neuronalen Netzen.

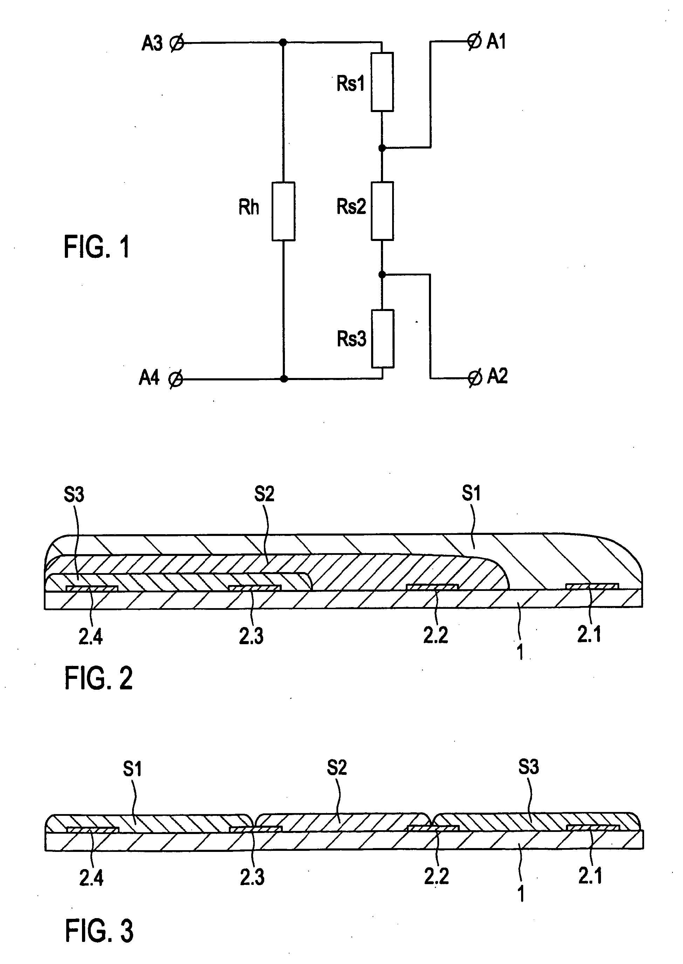

**Figur 1** erläutert die elektrische Schaltung der Sensoranordnung. Die drei Widerstände Rs1, Rs2 und Rs3 der Wirkschichten S sind in Reihe geschaltet und der Heiz- und Temperaturmesswiderstand Rh ist parallel zu dieser Reihenschaltung angeordnet. Hierzu wird ein Platinwiderstand verwendet, der bei 0°C einen elektrischen Widerstandswert von (5 ... 150) Ohm, bevorzugt von (10 ... 30) Ohm aufweist. Dieser Wert ist deutlich kleiner als die Summe der Widerstände Rs1, Rs2 und Rs3 der Wirkschichten S, die jeweils Werte von 1 kOhm bis 10 MOhm, bevorzugt von (10 bis 100) kOhm, aufweisen. Der Heizwiderstand Rh ist mit den beiden elektrischen Anschlüsse A3 und A4 verbunden. Die Anschlüsse A1 und A2 sind mit den elektrischen Verbindungsstellen der Widerstände Rs1-Rs2 und Rs2-Rs3 verbunden.

**Figur 2** zeigt eine Ausführung, bei der auf einem Substrat 1 vier Elektroden 2.1 ... 2.4 sowie drei sensitive Wirkschichten S1, S2 und S3 übereinander angeordnet sind.

Bei der in **Figur 3** dargestellten Sensoranordnung sind die drei sensitiven Wirkschichten S1, S2 und S3 nebeneinander angeordnet.

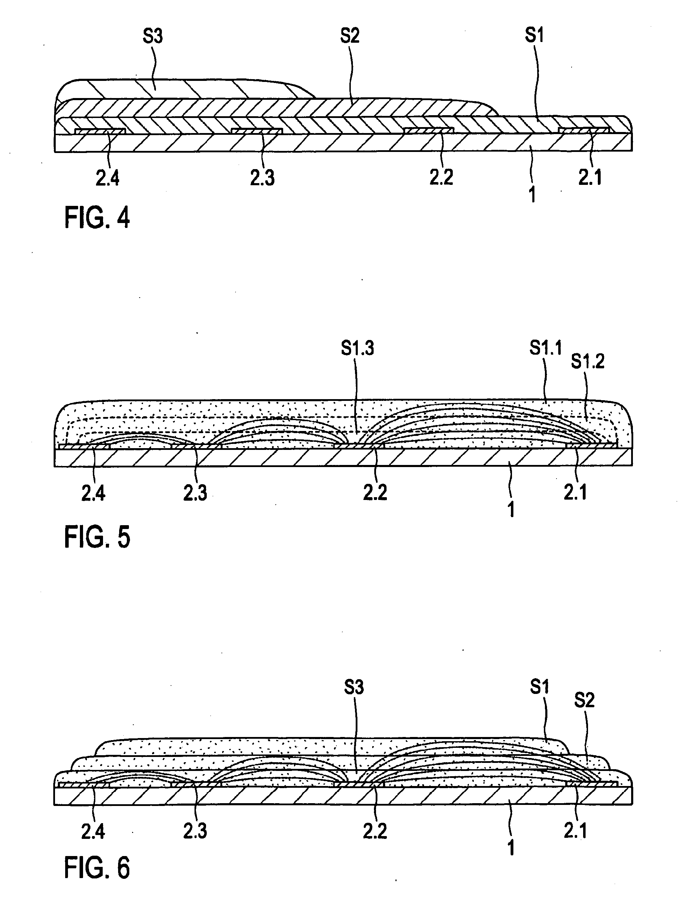

In **Figur 4** ist eine Ausführungsform gezeigt, bei der die sensitiven Wirkschichten S1, S2 und S3 versetzt übereinander angeordnet sind. Es ist auch möglich, dass das gassensitive Element aus einer Schicht besteht, die verschiedene Wirkbereiche aufweist.

Bei den in den **Figuren 5 und 6** dargestellten Varianten sind die Elektroden 2.1 ... 2.4 in unterschiedlichen Abständen voneinander auf dem Substrat 1 aufgebracht. Dadurch wird der zwischen benachbarten Elektroden 2 fließende Strom unterschiedlich tief in die Wirkschichten eindringen und damit durch unterschiedliche Wirkschichten oder unterschiedliche Wirkschichtbereiche verlaufen, so dass von außen eindringende Einflussgrößen, die zu Veränderungen der Eigenschaften der Wirkschichten führen, sich zwischen verschiedenen Elektrodenpaaren unterschiedlich auswirken. Derartige Veränderungen werden beispielsweise durch Verschmutzungen hervorgerufen. Dies führt zu einer relativen Veränderung der Widerstände, deren Ermittlung eine Aussage über die Veränderung (Verschmutzung) der Wirkschichten ermöglicht. Bei der in Figur 6 dargestellten Anordnung sind die Wirkschichten S1, S2 und S3 treppenförmig übereinander angeordnet, so dass dieser Effekt noch verstärkt wird. Das zu delektierende Gas diffundiert durch die porösen Wirkschichten hindurch und ruft dabei in unterschiedlichen Schichtbereichen unterschiedliche Reaktionen hervor. Je nach Anordnung der Schichtbereiche und der Elektroden können selektive Signale erzeugt werden. Damit ist es möglich, die Anordnung auf ein bestimmtes Zielgas abzustimmen.

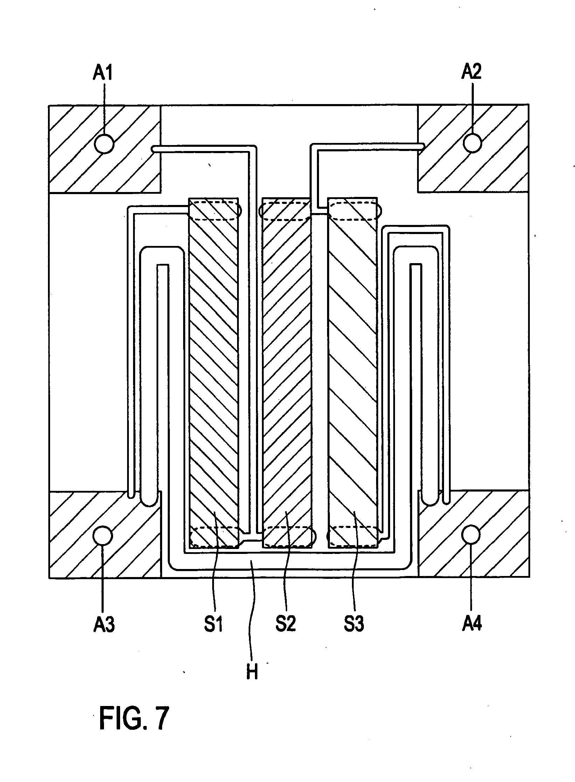

Die Draufsicht auf ein Chip mit der Sensoranordnung ist in **Figur 7** dargestellt. Der in diesem Beispiel dargestellte quadratisch ausgeführte Chip ist an seinen Ecken mit Kontaktstellen für die elektrischen Anschlüsse A1 ... A4 versehen, die mit den Elektroden 2.1 ... 2.4 verbunden sind. Die Elektroden 2.2 und 2.3 verbinden die Anschlüsse A1 bzw. A2 mit den Verbindungsstellen der sensitiven Wirkschichten S1-S2 bzw. S2-S3, während die Anschlüsse A3 und A4 mit den Enden des Heizers H und den Elektroden 2.1 bzw. 2.4 verbunden sind, welche zu den Enden der Wirkschichten S1 bzw. S3 führen.

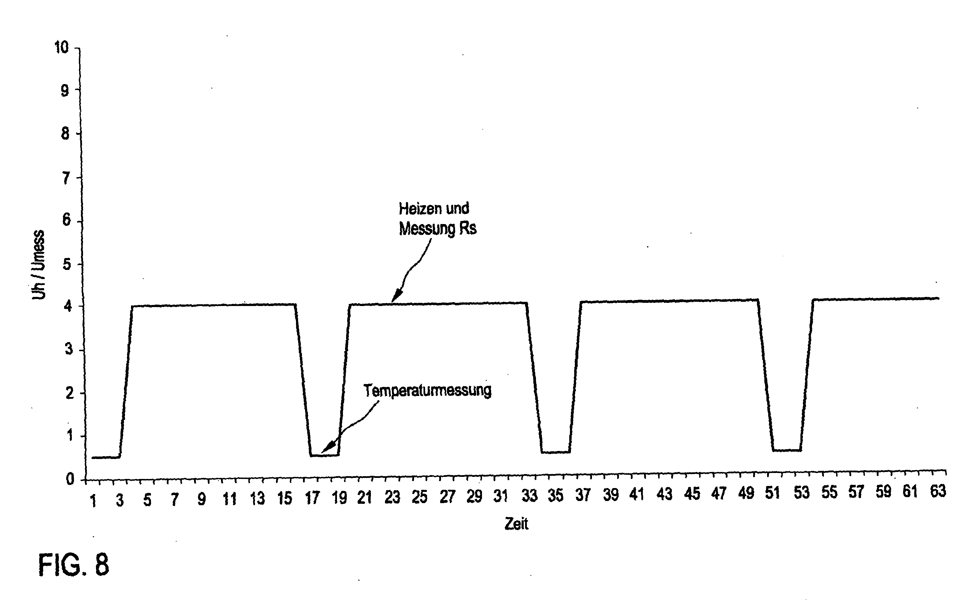

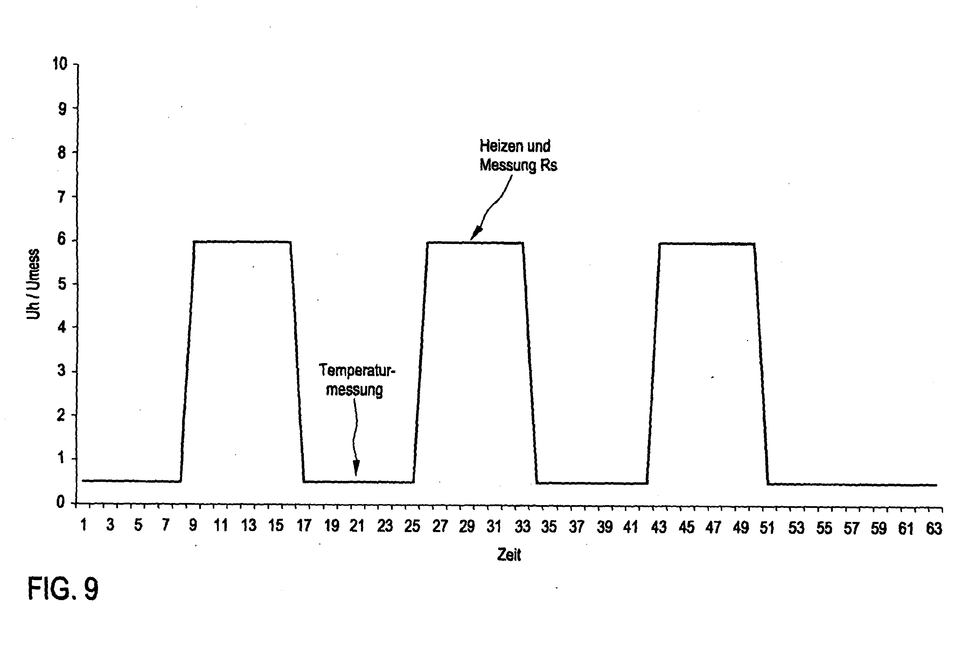

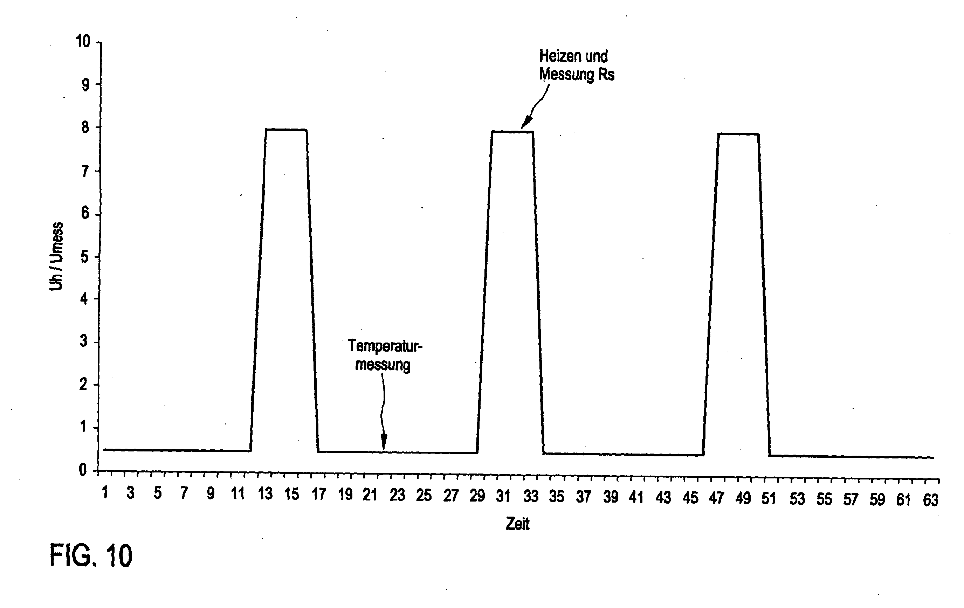

In den **Figuren 8 bis 10** sind Varianten für den zeitlichen Verlauf der an den Anschlüssen A3 und A4 des Heizers H anliegenden elektrischen Spannung dargestellt. Die Anordnung wird jeweils auf eine vorbestimmte Temperatur aufgeheizt, wobei aus einer Strom-Spannungs-Messung der Heizwiderstand Rh und damit die Temperatur der Anordnung bestimmt werden kann. Die Temperatur kann durch Wahl der Spannung und des Impulsverhältnisses eingestellt werden.
Dabei werden die Wirkschichten erhitzt, so dass die sich einstellenden Spannungsabfälle an den Wirkschichten S1, S2 und S3, die eine Funktion der Gaskonzentration darstellen, bei definierten Temperaturen ermittelt werden können. Die Spannungspotenziale an den elektrischen Verbindungsstellen, welche sich zwischen den Wirkschichten S1 und S2 sowie zwischen S2 und S3 befinden, können an den mit diesen Verbindungsstellen leitend verbundenen Anschlüssen A1 und A2 abgegriffen werden.

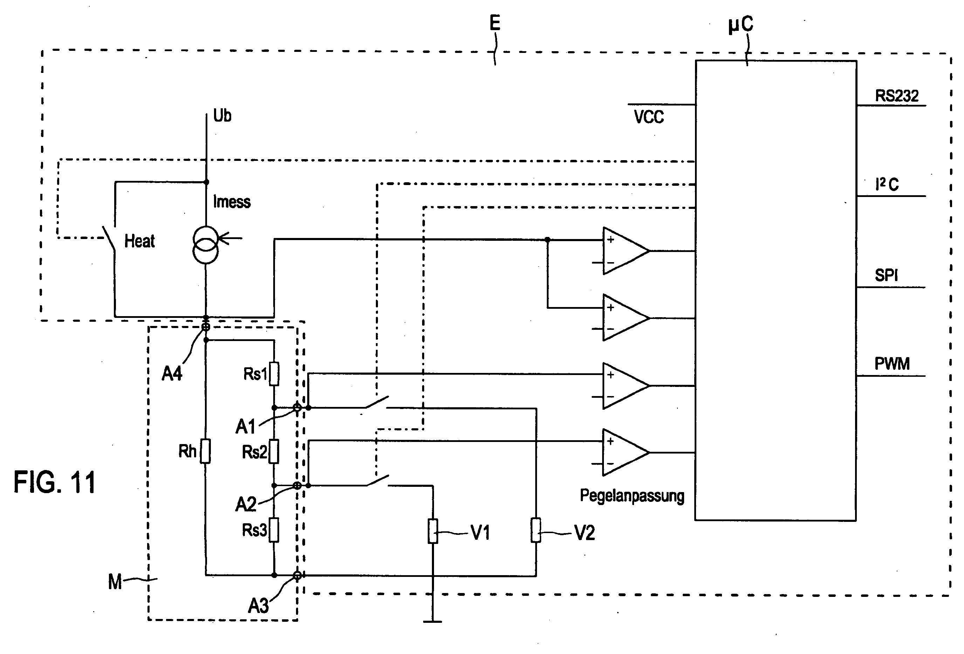

**Figur 11** erläutert das Schaltbild für eine Anordnung, bei der die Messanordnung M mit einer Auswerteeinheit E verbunden ist. Die Messanordnung M besteht aus einer Widerstandsparallelschaltung, bei der der Heiz- und Temperaturmesswiderstand Rh parallel zu den elektrischen Widerständen Rs1, Rs2 und Rs3 der Wirkschichten S geschaltet ist. Dabei ist der Heiz- und Temperaturmesswiderstand Rh über den Anschluss A4 mit der Spannungsquelle verbunden. Der durch den Heiz- und Temperaturmesswiderstand Rh fließende Heizstrom kann durch einen vom Mikrocontroller µC gesteuerten Schalter in der oben beschriebenen Weise an- und abgeschaltet werden. Die Anschlüsse A1, A2 und A3 verbinden die Auswerteeinheit E mit den elektrischen Widerständen Rs1, Rs2 und Rs3 der Wirkschichten S. Die Auswerteeinheit E enthält die Vergleichswiderstände V1 und V2, wobei V1 parallel zum Wirkschichtwiderstand Rs3 und V2 parallel zur Reihenschaltung von R2 und R3 geschaltet werden können. Das Anschalten der Vergleichswiderstände V1 und V2 wird gesteuert durch den Mikrocontroller µC, so dass der Auswerteeinheit E sowohl die Rohsignale, die aus den Spannungsabfällen der Wirkschichtwiderständen Rs1, Rs2 und Rs3 direkt gewonnen werden, als auch Signale aus einer Parallelschaltung der Vergleichswiderstände V1 und V2 mit elektrischen Widerständen R2 und R3 der Wirkschichten S gewonnen werden, zur Auswertung zur Verfügung stehen. Da die Werte der Vergleichswiderständen V1 und V2 bekannt sind, kann daraus eine Aussage über den Absolutwert der an den Wirkschichten S aktuell anliegenden Widerstandswerte Rs1, Rs2 und Rs3 gewonnen werden, mit der eine Aussage über das Vorhandensein und/oder die Konzentration eines bestimmten Gases getroffen werden kann.

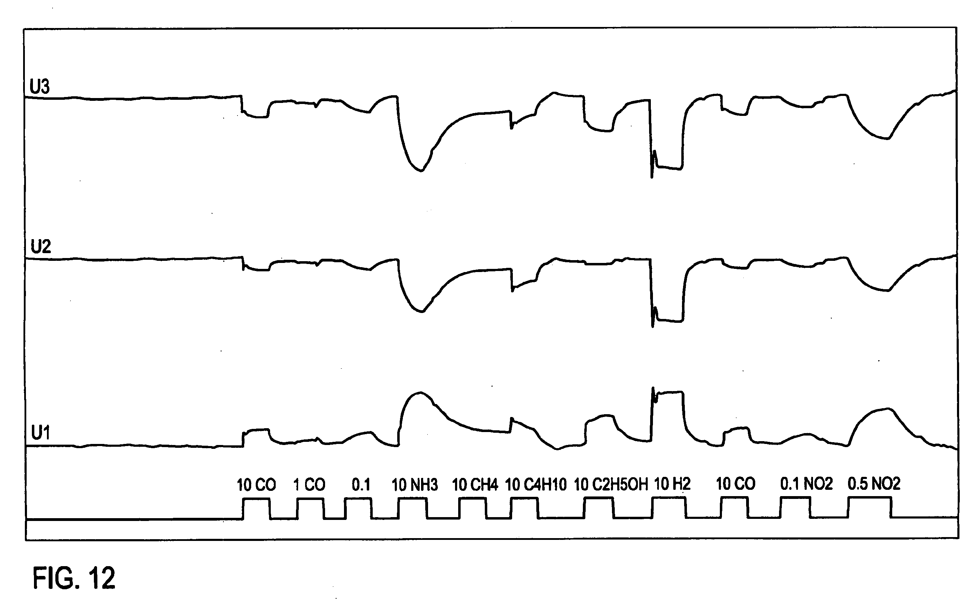

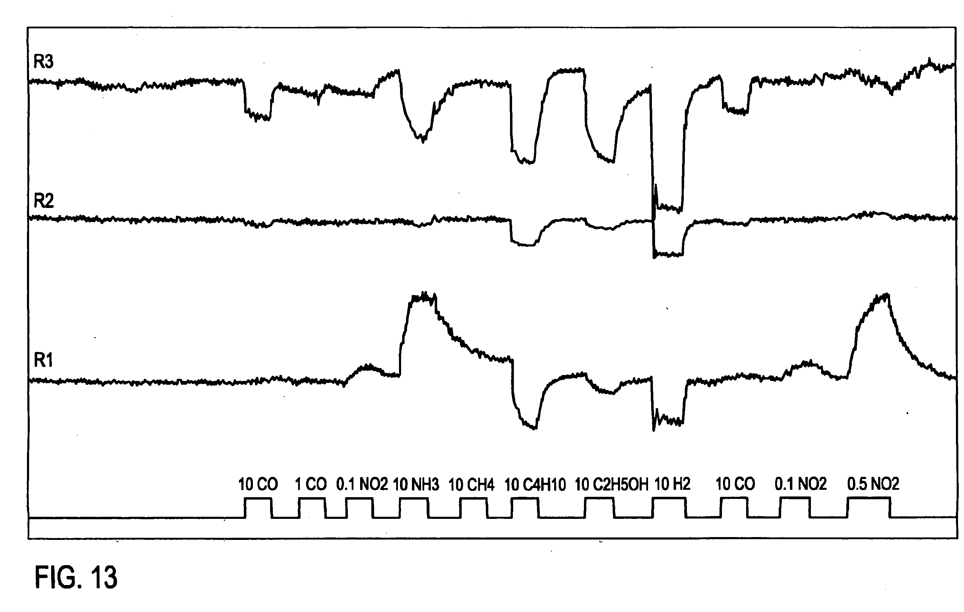

Die **Figuren 12** **und** **13** zeigen Messwertverläufe, die mit der erfindungsgemäßen Anordnung bei der Detektion unterschiedlicher, der Messanordnung M zeitlich nacheinander zugeführter Gasproben gewonnen wurden.
In Figur 12 ist der zeitliche Signalverlauf für die an den Anschlüssen A1, A2 und A3 gewonnenen Rohwerte U1, U2 und U3 dargestellt. Figur 13 zeigt den durch die Auswerteeinheit E unter Berücksichtigung der Vergleichswiderstände V1 und V2 ermittelte Verlauf der Realwerte R1, R2 und R3. Den Diagrammen kann entnommen werden, dass ohne Berücksichtigung der mit den Vergleichswiderständen V1 und V2 ermittelten Absolutwerten zwar eine Aussage über die Anwesenheit eines Gasgemisches möglich ist, durch die Auswertung der Absolutwerte ist darüber hinaus eine eindeutige Aussage über das Vorhandensein und gegebenenfalls die Konzentration eines bestimmten einzelnen Gases möglich.

### BEZUGSZEICHENLISTE

- 1: Substrat
- 2: Elektrode

- H: Heizer
- S: Wirkschicht
- A: elektrischer Anschluss
- Rh: Heizwiderstand
- Rs: Schichtwiderstand
- E: Auswerteeinheit
- V: Vergleichswideratand
- M: Messanordnung
- µC: Mikrocontroller

## Patentansprüche

1. Anordnung zur Detektion von Luftinhaltsstoffen, insbesondere zur Detektion von Luftinhaltsstoffen in Gebäuden und Fahrzeugen, bei der auf einem Substrat (1) mehrere für Gas sensitive Wirkschichten (S) sowie ein Heizer (H) angeordnet sind, die mit einer Auswerteeinheit (E) verbindbar sind, **dadurch gekennzeichnet, dass** die elektrischen Widerstände (Rs) von n Wirkschichten (S) elektrisch in einer Reihenschaltung verbunden sind, der Heizer (H) gleichzeitig ein Temperatursensor ist und parallel zu dieser Reihenschaltung geschaltet ist, wobei der elektrische Widerstand (Rh) des Heizers (H) deutlich kleiner ist als die Summe der elektrischen Widerstände (Rs) der Wirkschichten (S) und die Widerstände (Rh, Rs) über Elektroden (2) mit insgesamt n+1 elektrischen Anschlüssen (A1 ... Aₙ₊₁) so verbunden sind, dass der Heizer an zwei Anschlüssen (A3, A4) und n-1 weitere Anschlüsse (A1 ... Aₙ₋₁) mit Verbindungsstellen der Wirkschichten (S) verbunden sind.

2. Anordnung nach Anspruch 1, **dadurch gekennzeichnet, dass** die Anordnung drei unterschiedliche Wirkschichten (S) oder eine aus drei unterschiedlichen Schichtbereichen bestehende Wirkschicht (S) enthält, deren Verbindungsstellen mit zwei elektrischen Anschlüssen (A2, A3) mit der Auswerteeinheit (E) verbindbar sind, so dass die Sensoranordnung über insgesamt vier Anschlüsse verfügt.

3. Anordnung nach Anspruch 1 oder 2, **dadurch gekennzeichnet, dass** die Wirkschichten (S) aus SnO₂, WO₃ und anderen gasempfindlichen Metalloxiden einzeln oder gemischt bestehen.

4. Anordnung nach einem der vorhergehenden Ansprüche, **dadurch gekennzeichnet, dass** die Wirkschichten (S) versetzt übereinander angebracht sind.

5. Anordnung nach einem der vorhergehenden Ansprüche, **dadurch gekennzeichnet, dass** die Anordnung in einem Standardgehäuse angeordnet ist.

6. Anordnung nach einem der vorhergehenden Ansprüche, **dadurch gekennzeichnet, dass** die Auswerteeinheit (E) neuronale Netze enthält.

7. Anordnung nach einem der vorhergehenden Ansprüche, **dadurch gekennzeichnet, dass** die Abstände zwischen den auf dem Substrat (1) angebrachten Elektroden (E) unterschiedlich sind.

8. Anordnung nach einem der vorhergehenden Ansprüche, **dadurch gekennzeichnet, dass** die Anschlüsse (A1 ... Aₙ₊₁) mit einer Auswerteeinheit (E) verbunden sind, die mindestens einen Vergleichswiderstand (V) enthält, welcher parallel zu den Wirkschichtwiderständen (Rs1 ... Rsn) geschaltet werden kann, so dass an der Auswerteeinheit (E) sowohl Signale, die aus den an den Wirkschichtwiderständen (Rs1 ... Rsn) anliegenden Spannungen (U1 ... Un) direkt gewonnen werden, als auch Signale aus einem Vergleich dieser Signale mit Signalen, die unter Berücksichtigung mindestens eines Vergleichswiderstandes (V) gewonnen werden, anliegen.

9. Verfahren zur Detektion von Luftinhaltsstoffen, insbesondere zur Detektion von Luftinhaltsstoffen in Gebäuden und Fahrzeugen, durch Auswertung der elektrischen Parameter für Gas sensitive Wirkschichten (S), **dadurch gekennzeichnet, dass** ein Heizer (H) pulsierend so geheizt werden, dass eine vorbestimmte konstante Temperatur der Anordnung erreicht wird, wobei die Temperatur der Wirkschichten (S) durch Bestimmung des elektrischen Widerstandes des Heizers (H) erfasst wird, die an einer Reihenschaltung von Wirkschichten (S) auftretenden Spannungsabfälle ausgewertet werden und aus der Veränderung von elektrischen Widerständen (Rs) der Wirkschichten (S) oder der Wirkschichtbereiche das Vorhandensein und/oder die Konzentration bestimmter Gase ermittelt wird.

10. Verfahren nach Anspruch 9, **dadurch gekennzeichnet, dass** durch Vergleich von mit unterschiedlichen Elektrodenabständen ermittelten Wirkschichtwiderständen (Rs) das Driftverhalten der Wirkschichten (S) erfasst wird.

11. Verfahren nach Anspruch 9, **dadurch gekennzeichnet, dass** das Driftverhalten der Wirkschichten (S) durch Temperaturmodulation erfasst wird.

12. Verfahren nach Anspruch 9, **dadurch gekennzeichnet, dass** durch definierte Puls-Weiten-Modulation bei konstanter Temperatur das Driftverhalten der Wirkschichten erfasst wird.

13. Verfahren nach einem der Ansprüche 9 bis 12, **dadurch gekennzeichnet, dass** die Heizspannung einer Referenzschaltung und einem Differenzverstärker zugeführt wird.

14. Verfahren nach einem der Ansprüche 8 bis 13, **dadurch gekennzeichnet, dass** die an den einzelnen Wirkschichtwiderständen (Rs1 ... Rsn) auftretenden Spannungsabfälle mit Spannungsabfällen verglichen werden, die unter Berücksichtigung mindestens eines Vergleichswiderstandes (V) bekannter Größe auftreten und somit eine Aussage über den Absolutwert der an den Wirkschichten aktuell anliegenden Widerstandswerte ermöglicht, diese Werte einer elektronischen Auswerteeinheit (E) zugeführt werden, mit der eine Aussage über das Vorhandensein und die Konzentration eines bestimmten Gases gewonnen wird.

## Claims

1. Arrangement for the detection of air components, especially for the detection of air components in buildings and vehicles where (1) several gas sensitive active layers (S) and one heater (H) are arranged on a substrate and connectable to an analyzing unit (E), **characterized in that** the electrical resistors (Rs) of n active layers (S) are connected electrically in a series connection, the heater (H) also is a temperature sensor is and is connected in parallel to such series connection, while the electrical resistor (Rh) of the heater (H) has a considerably smaller value than the total of all electrical resistors (Rs) of the active layers (S) and the resistors (Rh, Rs) are connected via electrodes (2) to a total of n+1 electrical connections (A1 ... Aₙ₊₁) such that the heater is connected at two connections (A3, A4) and n-1 connections (A1 ... Aₙ₋₁) are connected to connecting points of the active layers (S).

2. Arrangement as claimed in claim 1 **characterized in that** the arrangement comprises three different active layers (S) or one active layer (S) consisting of three different layer areas, whose connecting points are connectable with two electrical connections (A2, A3) with the analyzing unit (E) such that the sensor arrangement has a total of four connections.

3. Arrangement as claimed in claim 1 or 2 **characterized in that** the active layers (S) consist of SnO₂, WO₃ and other gas-sensitive metal oxides either individually or mixed.

4. Arrangement as claimed in any preceding claim **characterized in that** the active layers (S) are arranged in offset positions one on top of the other.

5. Arrangement as claimed in any preceding claim, **characterized in that** the arrangement is located in a standard casing.

6. Arrangement as claimed in any preceding claim **characterized in that** the analyzing unit (E) contains neural networks.

7. Arrangement as claimed in any preceding claim **characterized in that** the distances between the electrodes (E) attached on the substrate (1) are different.

8. Arrangement as claimed in any preceding claim **characterized in that** the connections (A1 ... Aₙ₊₁) are connected to an analyzing unit (E) which contains at least one comparator resistor (V) which can be switched in parallel to the active layer resistors (Rs1 ... Rsn) such that both signals obtained directly form the voltages (U1 ... Un) applying to the active layer resistors (Rs1 ... Rsn), and signals obtained by a comparison of these signals with signals obtained by taking account of at least one comparator resistor (V) apply at the analyzing unit (E).

9. Procedure for the detection of air components, especially for the detection of air components in buildings and vehicles by analysing the electrical parameters for gas sensitive active layers (S) **characterized in that** one heater (H) is heated intermittently such that a predetermined constant temperature of the arrangement is attained where the temperature of the active layers (S) is acquired by determining the electrical resistance of the heater (H), where the voltage drops occurring at a series connection of active layers (S) are analysed, and changes in the electrical resistance values (Rs) of the active layers (S) or active layer areas are used to determine the presence and/or concentration of determined gasses.

10. Procedure as claimed in claim 9 **characterized in that** the drift behaviour of the active layers (S) is acquired by the comparison of active layer resistance values (Rs) determined at different electrode distances.

11. Procedure as claimed in claim 9 **characterized in that** the drift behaviour of the active layers (S) is acquired by temperature modulation.

12. Procedure as claimed in claim 9 **characterized in that** the drift behaviour of the active layers is acquired by defined pulse width modulation at a constant temperature.

13. Procedure as claimed in any claim 9 through 12 **characterized in that** the heating voltage is fed to a reference circuit and to a differential amplifier.

14. Procedure as claimed in any claim 8 through 13 **characterized in that** the voltage drops occurring at every active layer resistor (Rs1 ... Rsn) are compared to the voltage drops which occur taking account of at least one comparator resistor (V) of a known dimension, thus allowing a statement on the absolute value of the resistance values currently applying at the active layers, these values are fed to an electronic analyzing unit (E) which is used to obtain a statement on the presence or concentration of a determined gas.

## Revendications

1. Dispositif pour la détection de composants de l'air, en particulier pour la détection de composants dans l'air d'immeubles et de véhicules, où plusieurs couches actives (S) sensibles aux gaz ainsi qu'un filament chauffant (H) sont disposés sur un substrat (1) et reliables à une unité d'analyse (E), **caractérisé en ce que** les résistances électriques (Rs) de n couches actives (S) sont électriquement raccordées sur un montage en série, le filament chauffant (H) est simultanément un capteur de température et est connecté parallèlement à ce montage en série, la résistance électrique (Rh) du filament chauffant (H) étant sensiblement inférieure à la somme des résistances électriques (Rs) des couches actives (S) et les résistances (Rh, Rs) étant reliées par des électrodes (2) à un total de n+1 connexions électriques (A1 ... Aₙ₊₁), de telle manière que le filament chauffant soit raccordé sur deux connexions (A3, A4), et n-1 autres connexions (A1 ... Aₙ₋₁) à des points de raccordement des couches actives (S).

2. Dispositif selon la revendication 1, **caractérisé en ce que** le dispositif comprend trois couches actives (S) différentes, ou une seule couche active (S) composée de trois zones de couche différentes, dont les points de raccordement sont reliables à l'unité d'analyse (E) par deux connexions électriques (A2, A3), de telle manière que le dispositif de capteur dispose d'un total de quatre connexions.

3. Dispositif selon la revendication 1 ou 2, **caractérisé en ce que** les couches actives (S) se composent de SnO₂, WO₃ et d'autres oxydes métalliques sensibles aux gaz, isolément ou en mélange.

4. Dispositif selon l'une des revendications précédentes, **caractérisé en ce que** les couches actives (S) sont disposées en superposition alternée.

5. Dispositif selon l'une des revendications précédentes, **caractérisé en ce que** le dispositif est logé dans un carter standard.

6. Dispositif selon l'une des revendications précédentes, **caractérisé en ce que** l'unité d'analyse (E) comprend des réseaux neuronaux.

7. Dispositif selon l'une des revendications précédentes, **caractérisé en ce que** les espacements entre les électrodes (E) appliquées sur le substrat (1) sont différenciés.

8. Dispositif selon l'une des revendications précédentes, **caractérisé en ce que** les connexions (A1 ... Aₙ₊₁) sont reliées à une unité d'analyse (E) comprenant au moins une résistance de comparaison (V), laquelle peut être montée parallèlement aux résistances de couche active (Rs1 ... Rsn), de manière à délivrer à l'unité d'analyse (E) des signaux provenant directement des tensions (U1 ... Un) appliquées sur les résistances de couche active (Rs1 ... Rsn), ainsi que des signaux provenant d'une comparaison de ces signaux avec des signaux obtenus par prise en compte d'au moins une résistance de comparaison (V).

9. Procédé pour la détection de composants de l'air, en particulier pour la détection de composants dans l'air d'immeubles et de véhicules, par analyse des paramètres électriques pour des couches actives (S) sensibles aux gaz, **caractérisé en ce qu'**un filament chauffant (H) est chauffé par impulsions de manière à obtenir une température constante prédéfinie pour le dispositif, la température des couches actives (S) étant saisie par détermination de la résistance électrique du filament chauffant (H), **en ce que** les chutes de tension survenant sur un montage en série de couches actives (S) sont analysées, et **en ce que** la présence et/ou la concentration de gaz précis sont déterminées à partir de la variation des résistances électriques (Rs) des couches actives (S) ou des zones de couche active.

10. Procédé selon la revendication 9, **caractérisé en ce que** le comportement de dérive des couches actives (S) est saisi par comparaison de résistances de couches actives (Rs) déterminées avec des espacements différents entre électrodes.

11. Procédé selon la revendication 9, **caractérisé en ce que** le comportement de dérive des couches actives (S) est saisi par modulation de température.

12. Procédé selon la revendication 9, **caractérisé en ce que** le comportement de dérive des couches actives est saisi par modulation de largeur d'impulsion définie à température constante.

13. Procédé selon l'une des revendications 9 à 12, **caractérisé en ce que** la tension de chauffage est délivrée à un circuit de référence et à un amplificateur différentiel.

14. Procédé selon l'une des revendications 8 à 13, **caractérisé en ce que** les chutes de tension survenant sur les différentes résistances de couche active (Rs1 ... Rsn) sont comparées à des chutes de tension survenant par prise en compte d'au moins une résistance de comparaison (V) de grandeur connue, une information sur la valeur absolue des valeurs de résistance actuellement appliquées sur les couches actives pouvant ainsi être obtenue, ces valeurs étant délivrées à une unité d'analyse électronique (E) au moyen de laquelle une information est obtenue relativement à la présence et à la concentration d'un gaz précis.
